(19) Europäisches Patentamt
European Patent Office
Office européen des brevets

(11) Publication number: 0 312 995 B1

(12) # EUROPEAN PATENT SPECIFICATION

(45) Date of publication of patent specification: **05.08.92** (51) Int. Cl.$^5$: **A61K 7/11**

(21) Application number: **88117348.8**

(22) Date of filing: **18.10.88**

(54) **Hairdressing composition.**

(30) Priority: **21.10.87 JP 265991/87**

(43) Date of publication of application:
**26.04.89 Bulletin 89/17**

(45) Publication of the grant of the patent:
**05.08.92 Bulletin 92/32**

(84) Designated Contracting States:
**AT CH DE ES FR GB LI NL**

(56) References cited:
**EP-A- 0 168 574**
**DE-A- 3 212 861**

**PATENT ABSTRACTS OF JAPAN, vol. 10, no. 239 (C-367)[2295], 19th August 1986; & JP-A-61 72 706 (SHOWA DENKO K.K.) 14-04-1986**

**PATENT ABSTRACTS OF JAPAN, vol. 1, no. 91 (C-23), 24th August 1977, page 1916 C 77; & JP-A-52 51 035 (SHISEIDO K.K.) 23-04-1977**

(73) Proprietor: **KAO CORPORATION**
**14-10, Nihonbashi Kayabacho 1-chome**
**Chuo-ku Tokyo(JP)**

(72) Inventor: **Hikichi, Tadasu**
**No. 2218-76-2-39, Yonemoto**
**Yachiyo-shi Chiba(JP)**
Inventor: **Kure, Yuriko**
**No. 3-16-1-307, Higashisuna**
**Koto-ku Tokyo(JP)**

(74) Representative: **Hansen, Bernd, Dr.rer.nat. et al**
**Hoffmann, Eitle & Partner Patentanwälte Arabellastrasse 4 Postfach 81 04 20**
**W-8000 München 81(DE)**

EP 0 312 995 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a hairdressing composition comprising polyoxyalkylene compound(s) and crosslinked poly(acrylic acid/2,3-dihydroxypropyl acrylate) and/or crosslinked poly(acrylic acid/2-hydroxypropyl acrylate), which makes the hair moist, soft, smooth and nonsticky and exhibits a satisfactory hairdressing effect.

There are a number of hairdressing products including hair liquids, hair creams, set lotions and hair sprays which facilitate the formation of a hairstyle or maintain the same.

These hairdressing products comprise various oils and resins. However, a hairdressing composition comprising an excessive amount of an oil shows an intense oily feel, though it has, to some extent, a hairdressing effect. On the other hand, one comprising a large amount of a resin would give a stiff hairstyle, though it has a satisfactory hairdressing effect.

Recently, polyoxyalkylene compounds, such as polyoxypropylene butyl ether, have been employed in hairdressing composition since they have scarcely any oily or stiff feel, as described in JP-A-243012 (The term "JP-A" as used herein means an "unexamined published Japanese patent application"). However, these compounds can not always make the hair moist and smooth nor give a soft and natural hairstyle. That is, not only the hairdressing effect of a polyoxyalkylene compound, but also the stickiness thereof increases with an increase in the degree of polymerization of the same. On the other hand, not only the stickiness, but also the hairdressing effect thereof decreases with a decrease in the degree of polymerization thereof. Thus, the degree of polymerization of a polyoxyalkylene compound is to be selected by taking into consideration a balance between the hairdressing effect and stickiness.

DE-A- 3 212 861 discloses a hair setting lotion in which a propylene oxide and ethylene oxide adduct of a higher alcohol and an amphoteric polymer or a nonionic polymer are combined. Although this hair setting lotion is superior in terms of hair setting effect, this hair setting lotion cannot satisfy good feel of the hair and superior hair setting effect in simultaneously.

Accordingly, it has been required to enhance the hairdressing effect of a hairdressing composition without increasing the stickiness of the same.

### SUMMARY OF THE INVENTION

Under these circumstances, the present inventors have attempted to improve the abovedescribed disadvantages of polyoxyalkylene compounds. As a result, it has been found in the present invention that a hairdressing composition which has an improved hairdressing effect and makes the hair moist, soft, smooth and nonsticky can be obtained by blending polyoxyalkylene compound(s) with crosslinked poly(acrylic acid/2,3-dihydroxypropyl acrylate) and/or crosslinked poly(acrylic acid/2-hydroxypropyl acrylate).

Accordingly, the present invention relates to a hairdressing composition which comprises:

(A) a polyoxyalkylene compound(s) obtained by addition-polymerizing a monohydric or polyhydric alcohol and an alkylene oxide; and

(B) crosslinked poly(acrylic acid/2,3-dihydroxypropyl acrylate) and/or crosslinked poly(acrylic acid/2-hydroxypropyl acrylate).

Examples of the component (A), i.e., the polyoxyalkylene compound(s), used in the hairdressing composition of the present invention include those obtained by addition-polymerizing an aliphatic monohydric alcohol such as methanol, ethanol, butanol, octanol or lauryl alcohol, or a polyhydric alcohol such as propylene glycol, glycerol, 2-ethyl-2-hydroxymethyl-1,3-propanediol, mannitol or sorbitol, and an alkylene oxide. Examples of the alkylene oxide are those having 2 to 5 carbon atoms, preferably 2 to 4 carbon atoms, and specific examples thereof include ethylene oxide, propylene oxide, butylene oxide, etc. Among these alkylene oxides, propylene oxide, and a mixture of ethylene oxide and propylene oxide are preferable, and the former is still preferable. When a mixture of ethylene oxide and propylene oxide is used, it is preferable that the number of moles of the added ethylene oxide is 1/3 or less, preferably 1/4 or less, of the total added moles and that the components are block-polymerized. The total number of moles of the added alkylene oxide(s) is 2 to 100, preferably 6 to 100, still preferably 8 to 50.

Particularly preferred examples of the polyalkylene compound are those represented by general formulae (I) to (VI):

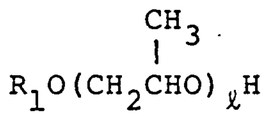

(I)

wherein $R_1$ represents an alkyl group having 1 to 22 carbon atoms, preferably 2 to 10 carbon atoms, and the average number of moles of the added propylene oxide ($\ell$) ranges from 10 to 100, preferably 10 to 50;

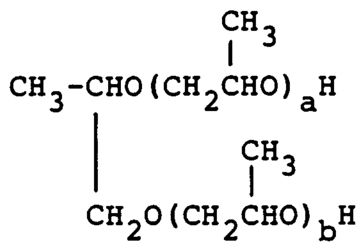

(II)

wherein the average number of moles of the added propylene oxide (a + b) ranges from 2 to 100, preferably 2 to 50;

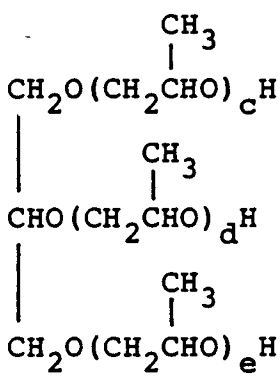

(III)

wherein the average number of moles of the added propylene oxide (c + d + e) ranges from 3 to 100, preferably 3 to 50;

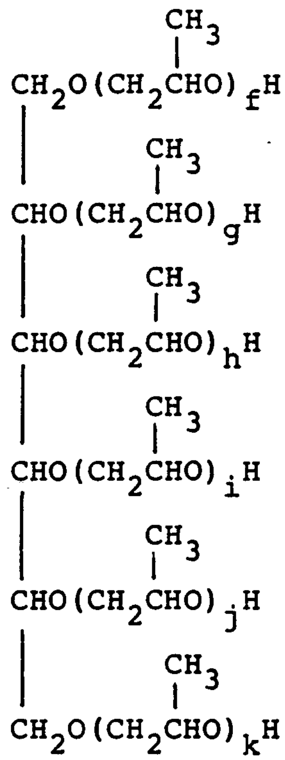

(IV)

wherein the average number of moles of the added propylene oxide (f + g + h + i + j + k) ranges from 2 to 100, preferably 2 to 50;

$R_1(EO)_m(PO)_nH$ (V)

wherein $R_1$ represents an alkyl group having 3 to 5 carbon atoms, EO represents ethylene oxide, PO represents propylene oxide, and the average number of moles of the added ethylene oxide and propylene oxide (m + n) ranges from 10 to 100, preferably 10 to 50; and

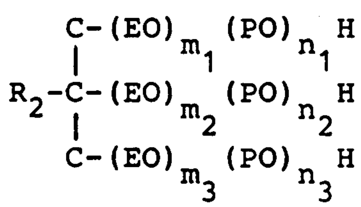

(VI)

wherein $R_2$ represents a hydrogen atom or an alkyl group having 1 to 4 carbon atoms, EO represents ethylene oxide, PO represents propylene oxide, and the average number of moles of the added ethylene oxide and propylene oxide ($m_1 + n_1 + m_2 + n_2 + m_3 + n_3$) ranges from 3 to 100, preferably 3 to 50.

Among these polyalkylene compounds, the compound represented by the general formula(IV) is particularly preferred.

Although the content of component (A) in the hairdressing composition of the present invention varies depending on the type of the hairdressing, it preferably ranges from 0.1 to 50%, preferably 3 to 30% (by weight, the same will apply hereinafter), based on the total weight of the composition.

Component (B) used in the hairdressing composition of the present invention, i.e., the crosslinked poly-(acrylic acid/2,3-dihydroxypropyl acrylate) and crosslinked poly(acrylic acid/2-hydroxypropyl acrylate), may be obtained respectively by 2,3-dihydroxypropyl-esterifying and 2-hydroxypropylesterifying a carboxyl group in a side chain of crosslinked polyacrylic acid as described, for example, in JP-A-61-72706. The degree of the esterification, which is represented by a ratio (mol%) of the esterified carboxyl group per the carboxyl group in a side chain of the crosslinked polyacrylic acid, preferably ranges from 1 to 50%. The crosslinked polyacrylic acid may be obtained by polymerizing acrylic acid and a crosslinking agent in an

organic solvent such as benzene. As the crosslinking agent, a monomer containing two or more vinyl group may be employed. Specific examples of the crosslinking agent include divinylbenzene, ethylene glycol diacrylate, ethylene glycol dimethacrylate, trimethylol propane acrylate. In order to obtain a hairdressing composition showing a nonsticky and refreshing feel upon use, it is preferable to use the crosslinking agent within a range of 0.01 to 10% more preferably 0.1 to 5%, based on 100% by weight of a monofunctional monomer.

In order to obtain a hairdressing having an excellent moistening effect, it is preferable that the molar ratio of the acrylic acid unit to the 2,3-dihydroxypropyl acrylate or 2-hydroxypropyl acrylate unit in the crosslinked poly(acrylic acid/2,3-dihydroxypropyl acrylate) or crosslinked poly(acrylic acid/2-hydroxypropyl acrylate) ranges from 99.5 - 30 : 0.5 - 70, more preferably from 99 - 50 : 1 - 50.

The crosslinked poly(acrylic acid/2,3-dihydroxypropyl acrylate) and/or crosslinked poly(acrylic acid/2-hydroxypropyl acrylate) may be incorporated into the hairdressing composition within a range of 0.01 to 10%, preferably 0.1 to 3%, based on the total weight of the hairdressing composition.

The hairdressing composition according to the present invention can be produced, for example, by the following procedure.

Component (A), component (B), an aqueous component, and water are mixed at a temperature of about 10 to 80°C to prepare a composition I. Separately, an oil component and an emulsifying agent are added to the water heated to 30 to 80°C to prepare an emulsion II. The obtained emulsion II is gradually added to the obtained composition I under stirring. After cooling to a room temperature (15 to 25°C), a component having a low heat stability such as a lower alcohol, a perfume, etc. is added thereto to obtain a hairdressing composition as a desired product.

The hairdressing composition of the present invention may be formulated into various forms including a hair liquid, a hair cream, a set lotion and a hair spray by blending the essential components (A) and (B), optionally together with various additives as will be described hereinafter, with a solvent such as water, a lower alcohol or a water/lower alcohol mixture in a conventional manner. When it is to be formulated into an aerosol, it is preferable to use a propellant selected from among chlorofluoroalkane propellants, such as chlorofluoroalkane propellants including trichloromonofluoromethane, dichlorodifluoromethane and dichlorotetrafluoroethane, liquified petroleum gas, dimethyl ether and mixtures thereof. This propellant is preferably incorporated in such a manner as to give an internal pressure within an aerosol can of 2.0 to 4.0 $kg/m^2 \cdot G$. A mist-type aerosol hairdressing composition may preferably contain 50 to 80% or more of the propellant, while a foam-type aerosol hairdressing composition may preferably contain 10 to 30% or less of the same.

In addition to the above-mentioned essential components, the hairdressing composition of the present invention may further contain various cosmetic fats and oils selected from among, for example, glycerides such as castor oil, cacao oil, mink oil, avocado oil and olive oil; waxes such as beeswax, sperm whale, lanolin and carnauba wax; alcohols such as cetyl alcohol, oleyl alcohol, lauryl alcohol, stearyl alcohol, propylene glycol, polypropylene glycol and glycerol; esters such as isopropyl myristate, hexyl laurate, cetyl lactate, propylene glycol monostearate, oleyl oleate, hexadecyl 2-ethylhexanoate and octyldodecyl myristate; and silicone derivatives such as dimethyl polysiloxane, methylphenyl polysiloxane, polyether-denatured silicone oil, epoxy-denatured silicone oil, amino-denatured silicone oil and alkyl-denatured silicone oils, so long as the effects of the present invention are not inhibited thereby. The hairdressing composition of the present invention may further contain an emulsifier to thereby stabilize the emulsification. In addition, it may further contain a perfume and/or a colorant to thereby elevate the commercial value. Furthermore, it may contain a preservative or an antioxidant to thereby prevent deterioration of the composition upon storage.

As described above, the present invention enables one to obtain an excellent hairdressing, which has a high hairdressing effect and makes the hair moist, soft, smooth and nonsticky, by blending the above-mentioned components (A) and (B).

The present invention is now illustrated in greater detail with reference to Examples. In these Examples, the properties of each hairdressing product were evaluated in the following manner.

(1) Hairdressing effect:

A bundle of hair weighing 4 g was moistened with water and 1 ml of hairdressing composition was applied thereon. After combing and drying with a hot air stream for 30 minutes, the condition of the hair bundle was evaluated according to the following criterion.

o : the hair bundle was neatly dressed, suggesting that the hairdressing effect was high; and
x : the hair was disheveled, suggesting that the hair-dressing effect was poor.

(2) Stickiness and moistness:

A bundle of hair treated in the same manner as the one described in (1) was organoleptically evaluated by ten experienced panelists according to the following criterion.

The results were expressed in the means of the panelists.

+2 : very good,
+1 : good,
0 : moderate,
-1 : poor, and
-2 : very poor.

EXAMPLE 1

Hairdressings of the following compositions were prepared, and the hairdressing effect, stickiness and moistness of each product were evaluated.

The results are shown in Table 1 below.

| Composition | Amount(%) |
|---|---|
| polyoxypropylene glyceryl ether (in formula (III), c+d+e is 15) | 0 or 3.0 |
| crosslinked poly(acrylic acid/2,3-di-hydroxypropyl acrylate) (degree of esterification: 5%) | see Table 1 |
| 95% (v/v) ethanol | 5.0 |
| water | balance |
| | Total 100.0 |

**Table 1**

| Composition (%) | | Evaluation | | |
|---|---|---|---|---|
| Polyoxypropylene glyceryl ether | Crossliked poly(acrylic acid/2,3-dihydroxypropyl acrylate) | Hairdressing effect | Stickiness | Moistness |
| Invention | | | | |
| 3.0 | 0.05 | o | +1.2 | +1.2 |
| 3.0 | 0.50 | o | +1.6 | +1.8 |
| 3.0 | 1.00 | o | +1.8 | +1.8 |
| Composition | | | | |
| 3.0 | 0 | x | -1.0 | -1.4 |
| - | 1.00 | x | -0.6 | +0.4 |

EXAMPLE 2

Hairdressing of the following composition were prepared, and the hairdressing effect, stickiness and moistness of each product were evaluated.

The results are shown in Table 2 below.

| Composition | Amont (%) |
|---|---|
| polyoxypropylene compound | 5.0 |
| crosslinked poly(acrylic acid/2-hydroxypropyl acrylate) (degree of esterification: 8.5%) | 0 or 0.5 |
| polyoxyethylene (20) lauryl ether | 0.5 |
| 95% (v/v) ethanol | 5.0 |
| water | balance |
| | Total 100.0 |

Table 2

| | Invention | | | Comparison | | |
|---|---|---|---|---|---|---|
| Composition (%) | | | | | | |
| polyoxypropylene glycol ether (in formula (II), a+b=6) | 5.0 | - | - | 5.0 | - | - |
| polyoxypropylene glyceryl ether (in formula (III), c+d+e=18) | - | 5.0 | - | - | 5.0 | - |
| polyoxypropylene sorbitol ether (in formula (IV), f+g+h+i+j+k=10) | - | - | 5.0 | - | - | 5.0 |
| crosslinked poly(acrylic acid/2-hydroxypropyl acrylate) | 0.5 | 0.5 | 0.5 | - | - | - |
| Evaluation | | | | | | |
| Hairdressing effect | o | o | o | x | x | x |
| Stickiness | +1.2 | +1.0 | +1.6 | -1.1 | -1.3 | -0.9 |
| Moistness | +1.2 | +1.2 | +1.8 | -1.2 | -1.2 | -1.4 |

EXAMPLE 3

Hairdressings of the following composition were prepared, and the hairdressing effect, stickiness and moistness of each product were evaluated.

The results are shown in Table 3 below.

| Composition | Amount (%) |
|---|---|
| polyoxypropylene sorbitol ether (in formula (IV), f + g + h + i + j + k = 9) | 0 or 10.0 |
| crosslinked poly(acrylic acid/2-hydroxypropyl acrylate) (degree of esterification: 8.5%) | 0 or 0.5 |
| Softanol 90 | 1.0 |
| perfume | 0.5 |
| 95% (v/v) ethanol | 5.0 |
| water | balance |
| | Total 100.0 |

Table 3

| | Invention | Comparison | |
|---|---|---|---|
| Composition (%) | | | |
| polyoxypropylene sorbitol ether | 10.0 | - | 10.0 |
| crosslinked poly(acrylic acid/2-hydroxypropyl acrylate) | 5.0 | 5.0 | - |
| Evaluation | | | |
| Hairdressing effect | o | x | x |
| Stickiness | +1.2 | -1.0 | -1.6 |
| Moistness | +1.8 | +0.6 | -1.2 |

From the results shown in Tables 1 to 3, it can be seen that the hairdressing composition comprising component (A) and component (B) according to the present invention has a high hairdressing effect and makes the hair moist, soft, smooth and nonsticky.

## Claims

1. A hairdressing composition comprising:
   (A) a polyoxyalkylene compound(s) obtained by addition-polymerizing a monohydric or polyhydric alcohol and an alkylene oxide; and
   (B) crosslinked poly(acrylic acid/2,3-dihydroxypropyl acrylate) and/or crosslinked poly(acrylic acid/2-hydroxypropyl acrylate).

2. The hairdressing composition as claimed in Claim 1, wherein said monohydric alcohol is selected from the group consisting of methanol, ethanol, butanol, octanol, and lauryl alcohol and said polyhydric alcohol is selected from the group consisting of propylene glycol, glycerol, 2-ethyl-2-hydroxymethyl-1,3-propanediol, mannitol and sorbitol.

3. The hairdressing composition as claimed in Claim 1, wherein said alkylene oxide has 2 to 5 carbon atoms.

4. The hairdressing composition as claimed in Claim 1, wherein said component (A) is employed in an amount of from 0.1 to 50% by weight based upon the total weight of the composition.

5. The hairdressing composition as claimed in Claim 1, wherein said component (B) is employed in an amount of from 0.01 to 10% by weight based upon the total weight of the composition.

6. The hairdressing composition as claimed in Claim 1, wherein the molar ratio of the acrylic acid unit to the 2,3-dihydroxypropyl acrylate or 2-hydroxypropyl acrylate unit in the crosslinked poly(acrylic acid/2,3-dihydroxypropyl acrylate) and the crosslinked poly(acrylic acid/2-hydroxypropyl acrylate), respectively, ranges from 99.5 - 30 : 0.5 - 70.

7. The hairdressing composition as claimed in Claim 6, wherein the molar ratio of the acrylic acid unit to the 2,3-dihydroxypropyl acrylate or 2-hydroxypropyl acrylate unit in the crosslinked poly(acrylic acid/2,3-dihydroxypropyl acrylate) and the crosslinked poly(acrylic acid/2-hydroxypropyl acrylate), respectively, ranges from 99 - 50 : 1 - 50.

## Revendications

1. Composition pour le coiffage ou la mise en forme des cheveux, cette composition comprenant :
   (A) un ou des composé(s) polyoxyalkylénique(s), que l'on obtient par polymérisation par addition d'un monoalcool ou d'un polyalcool et d'un oxyde d'alkylène ; et
   (B) du poly(acide acrylique/acrylate de 2,3-dihydroxypropyle) réticulé et/ou du poly(acide acrylique/acrylate de 2-hydroxypropyle) réticulé.

2. Composition pour le coiffage ou la mise en forme des cheveux, telle que revendiquée à la revendication 1, dans laquelle ledit monoalcool est choisi dans l'ensemble consistant en le méthanol, l'éthanol, le butanol, l'octanol et l'alcool laurylique, et ledit polyalcool est choisi dans l'ensemble consistant en le propylèneglycol, le glycérol, le 2-éthyl-2-hydroxyméthyl-1,3-propanediol, le mannitol et le sorbitol.

3. Composition pour le coiffage ou la mise en forme des cheveux, telle que revendiquée à la revendication 1, dans laquelle ledit oxyde d'alkylène comporte 2 à 5 atomes de carbone.

4. Composition pour le coiffage des cheveux, telle que revendiquée à la revendication 1, dans laquelle ledit composant (A) est présent en une quantité de 0,1 à 50 % en poids, sur la base du poids total de la composition.

5. Composition pour le coiffage des cheveux, telle que revendiquée à la revendication 1, dans laquelle ledit composant (B) est présent en une quantité de 0,01 à 10 % en poids, sur la base du poids total de la composition.

6. Composition pour le coiffage des cheveux, telle que revendiquée à la revendication 1, dans laquelle le rapport molaire du motif acide acrylique au motif acrylate de 2,3-dihydroxypropyle ou acrylate de 2-hydroxypropyle se situe, dans le poly(acide acrylique/acrylate de 2,3-dihydroxypropyle) réticulé et dans le poly-(acide acrylique/acrylate de 2-hydroxypropyle) réticulé, respectivement, entre 99,5 et 30 : 0,5-70.

**7.** Composition pour le coiffage des cheveux, telle que revendiquée à la revendication 6, dans laquelle le rapport molaire du motif acide acrylique au motif acrylate de 2,3-dihydroxypropyle ou acrylate de 2-hydroxypropyle dans le poly(acide acrylique/acrylate de 2,3-dihydroxypropyle) réticulé et dans le poly-(acide acrylique/acrylate de 2-hydroxypropyle) réticulé, respectivement, se situe entre 99 et 50 : 1-50.

## Patentansprüche

**1.** Frisierzusammensetzung, umfassend:
(A) eine Polyoxyalkylenverbindung(en), erhalten durch Additionspolymerisation eines ein- oder mehrwertigen Alkohols und eines Alkylenoxids; und
(B) vernetztes Poly(arylsäure/2,3-dihydroxypropylacrylat) und/oder vernetztes Poly(acrylsäure/2-hydroxypropylacrylat).

**2.** Frisierzusammensetzung gemäß Anspruch 1, in welcher der einwertige Alkohol ausgewählt ist aus der Gruppe bestehend aus Methanol, Ethanol, Butanol, Octanol und Laurylalkohol und der mehrwertige Alkohol ausgewählt ist aus der Gruppe bestehend aus Propylenglykol, Glycerin, 2-Ethyl-2-hydroxymethyl-1,3-propandiol, Mannit und Sorbit.

**3.** Frisierzusammensetzung gemäß Anspruch 1, in welcher das Alkylenoxid 2 bis 5 Kohlenstoffatome hat.

**4.** Frisierzusammensetzung gemäß Anspruch 1, in welcher die Komponente (A) in einer Menge von 0,1 bis 50 Gew.-%, bezogen auf das Gesamtgewicht der Zusammensetzung, angewendet wird.

**5.** Frisierzusammensetzung gemäß Anspruch 1, in welcher die Komponente (B) in einer Menge von 0,01 bis 10 Gew.-%, bezogen auf das Gesamtgewicht der Zusammensetzung, angewendet wird.

**6.** Frisierzusammensetzung gemäß Anspruch 1, in welcher das Molverhältnis der Acrylsäureeinheit zu der 2,3-Dihydroxypropylacrylat- oder 2-Hydroxypropylacrylateinheit in dem vernetzten Poly(acrylsäure/2,3-dihydroxypropylacrylat)- bzw. dem vernetzten Poly(acrylsäure/2-hydroxypropylacrylat) im Bereich von 99,5 - 30 : 0,5 - 70 liegt.

**7.** Frisierzusammensetzung gemäß Anspruch 6, in welcher das Molverhältnis der Acrylsäureeinheit zu der 2,3-Dihydroxypropylacrylat)- oder 2-Hydroxypropylacrylateinheit in dem vernetzten Poly(acrylsäure/2,3-Dihydroxypropylacrylat) bzw. dem vernetzten Poly)acrylsäure/2-hydroxypropylacrylat) im Bereich von 99 - 50 : 1 - 50 liegt.